# EUROPEAN PATENT SPECIFICATION

(11) **EP 0 263 978 B1**
(45) Date of publication and mention of the grant of the patent: **25.05.1994**
(21) Application number: 87113268.4
(22) Date of filing: 10.09.1987
(51) Int. Cl.: G01N 33/53, G01N 33/556, G01N 33/49, G01N 33/52, G01N 33/543

(54) **Method and apparatus for assaying whole blood**
Verfahren und Apparat zur Prüfung von Gesamtblut
Méthode et dispositif pour tester le sang intégral

(30) Priority: 10.09.1986 US 905856
(43) Date of publication of application: 20.04.1988
(73) Proprietor: IDEXX LABORATORIES, INC., Westbrook, ME 04092 (US)
(72) Inventor: Tonelli, Quentin J., Portland, ME 04103 (US)
(74) Representative: Bardehle, Heinz, Dipl.-Ing.

(56) References cited:
- EP-A- 0 190 488
- WO-A-79/01120
- FR-A- 2 344 842
- US-A- 3 963 119
- US-A- 3 972 812
- CHEMICAL ABSTRACTS, vol. 102, 21 January 1985, Columbus, OH (US); V. LIPOVAC, p. 484, no. 22174m#

## Description

### Background of the Invention

This invention relates to the performance of immunoassays on whole blood.

Immunoassays are carried out to detect a particular antigen, hapten, or antibody in blood.

Since red blood cells interfere with such assays,
they are normally removed from the blood prior to performing the assay,
e.g., by centrifugation or filtration.

The resulting plasma solution can then be used in any standard immunoassay, cf. e.g.
**US - A - 4 136 161** (GERBER et al.),
which describes a colorimetric method of detection of bindable substances such as antigens, using chromogenic agents such as tetramethyl benzidine.

The state of the art for assays of whole blood involves
the following disadvantages :
a) tolerating the presence of red blood cells, thus
   limiting the flexibility of assaying and detection techniques;
b) more complex separations, using
   chemical binding reagents or
   mechanical techniques such as centrifugation;
c) attempts to filter, with attendant problems of
   clogging or
   slowness or
   undesired passage of red cells.

**WO - A - 79 / 01120** (GOVERNMENT ... UNITED STATES ...) discloses
a laminar flow device
for separating red blood cells from plasma, i.e.
a flow regime having two forces :
a force perpendicular to the membrane to force the plasma through; and
a force tangential to the membrane to prevent blood cell/membrane clogging.

**FR - A - 2 344 842** (ORTHO DIAGNOSTICS INC.) discloses
a method for stabilizing erythrocytes.

Incidentally, the treatment of blood with isotonic and hypertonic salt solutions is known,
**FR - A - 2 344 842** (ORTHO DIAGNOSTICS INC.), and
**EP - A - 0 190 488** (BECTON DICKINSON & CO.).

### Summary of the Invention

In general, the invention features a method of assaying a sample of whole blood for one member of a specific binding pair, involving treating the sample with a salt to alter the red blood cells in the sample to decrease their ability to pass through filter media, exposing the resultant sample to a filter which retains the red blood cells but allows the passage therethrough of filtrate containing the member of the specific binding pair being assayed, and measuring the member of the filtrate. As used herein, the term "specific binding pair" means any pair of molecules which bind to each other but do not bind to a significant degree to other molecules; examples of such pairs are antigens and antibodies specific therefore.

It is believed that the salt causes the red blood cells in the sample to exhibit a change in physical properties, e.g., deformability, so that they are unable to pass through filter media which would have permitted their passage prior to treatment with the salt. A filter having an average pore size small enough to retain untreated red blood cells would become clogged and prevent passage of liquid as well. According to the invention, a filter can be used which has an average pore size (nominal size 0.3-0.6 µm (microns)) large enough to prevent clogging, but which still retains the treated red blood cells.

In preferred embodiments of the method, the step of treating the sample with a salt involves mixing the sample with a hypertonic solution to form a dilute solution, and following the exposing of the sample to the filter, the filtrate is deposited on a solid support on which the measuring of the member of the specific binding pair is carried out; preferably, the solid support and the filter are in contact with one another when the sample is exposed to the filter.
Alternatively, the filtrate is removed from the filtration device and placed in contact with a measuring device comprising a separate solid support on which measuring is carried out. Preferably, the hypertonic solution contains a salt of ionic strength of at least 0.5 M.

The method of the invention is preferably carried out using a kit composed of a first container holding a hypertonic solution, a second container for holding the hypertonic solution and mixing it with the sample, a filter positioned with respect to the second container so as to permit the mixture of the sample and the hypertonic solution to pass from the container through the filter, such filter being capable of retaining the treated red blood cells in the sample, and means e.g., a solid support, for collecting the sample after it has passed through the filter.

In preferred embodiments of the kit, the filter is flexible, and the kit comprises means to hold the filter in intimate contact with the solid support. The solid support comprises a surface bound to one of the components of the specific binding pair, e.g., an antigen, a virus, or an antibody. The filter is positioned within a holder, which may comprise a fluid chamber having sloping sides to improve flow consistency. The filter is sealed to the holder. The kit includes a filter holder having an annular member comprising an annular exterior wall and an annular interior wall, attached by a connecting member; the exterior wall and the interior wall are spaced apart a distance selected to form a cavity for receiving an co-operatively shaped seating member connected to the measuring device. Either the interior wall or the said exterior wall comprises a surface irregularity along the cavity to seat the measuring device, and the surface irregularity comprises means for venting air as said filter device is seated on said measuring device.

In another aspect, the invention features the above-described filter device. The filter device preferably includes means to impart tension to the filter, e.g., an annular screen positioned to contact the filter.

The invention allows the rapid and simple assay of whole blood samples for any immunologically detectable compound, without prior removal of red blood cells.

Other features and advantages of the invention will be apparent from the following description of the preferred embodiments thereof, and from the claims.

### Description of the Preferred Embodiment

Fig. 1 is a diagrammatic representation of an assay filter assembly of the invention.

Fig. 2 is a section of an alternative filter assembly.

### Structure

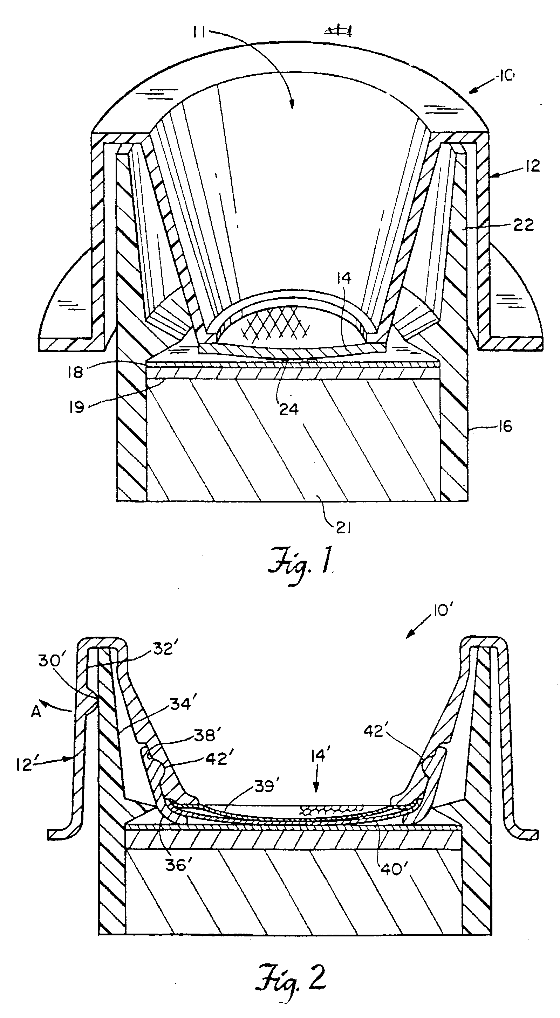

Referring to the Fig. 1, cylindrical membrane assembly 10 includes filter holder 12 fitted with filter 14 to form fluid chamber 11, and an assay membrane holder 16 fitted with assay membrane 18. Membrane holder 16 and filter holder 12 may be constructed of any suitable nonporous material, for example, PVC.

Filter holder 12 has an annular groove which mates with annular ridge 22 on assay membrane holder 16 so that filter 14 is positioned in intimate and homogeneous contact with assay membrane 18, which is supported by porous support disc 19, which in turn overlies absorbent bed 21. Assay membrane 18 bears immunologically reactive material 24 which consists, for example, of latex microspheres 1 micron in diameter coated with a suitable immunologically reactive compound, for example, an antibody, or of a bioreactive zone prepared by binding the immunologically reactive compound directly to a solid support consisting of either an activated surface or an inert surface capable of irreversibly binding the immunologically reactive compound.

Assembly 10 is of a diameter suitable for holding membrane 18, for example, 1 cm. Membrane 18 and filter 14 are attached to their respective holders by glue, or other means which prevents leakage between the holder and membrane.

Filter 14 is capable of retaining red blood cells in a mixture of whole blood and salt while allowing passage therethrough of red blood cell-free liquid. Filter 14 can e.g., be a filter having fibers lying on top of one another with a thickness of 1/4 to 1/2 mm and a nominal pore size rating of 0.4 microns, for example, a glass AE filter (Gelman). Assay membrane 18 can be any standard assay membrane, e.g., a glass AE filter.

A second filter embodiment is shown in Fig. 2. The features of filter membrane assembly 10ʹ are generally the same as those shown in Fig. 1, and corresponding primed members are used to designate those features in Fig. 2. Filter membrane assembly 10ʹ also includes a protrusion 30ʹ on the inside of the outer wall 32ʹ of filter holder 12ʹ. The depth of outer wall 32ʹ and support wall 34ʹ are carefully selected so that filter 14ʹ is in intimate contact with porous support disc to support capillary action.

In Fig. 2, filter 14ʹ is a glass fibre filter disc sealed to assembly 10ʹ by snap ring 36ʹ which has an annular protrusion 42ʹ that fits within an indentation in the inner wall 38ʹ of holder 12ʹ. A polypropylene screen 39ʹ (150 mm) which includes a 1/4ʺ central opening, is molded into filter 14ʹ. The screen assures that the glass fiber filter disc retains the desired convex shape. The convex form of the screen transfers a kinetic advantage to the weaker glass fiber disc. As the center point of the filter contacts the assay membrane, positive tension is generated as the rest of the glass fiber filter is seated against the assay membrane. This tension ensures an intimate contact between both surfaces. As the required tension is slight, material has been removed from the center of the screen to prevent stressing of the glass fiber filter. The dimensions of the filter holder have been carefully determined to mate with the solid support. Specifically, support wall 34ʹ and filter support 12ʹ form a close fit. There is a risk that an increase in air pressure between the glass fiber filter and assay membrane during seating could distort the shape of the filter. Additionally, any unexpelled air could remain trapped between the surfaces resulting in non-intimate contact. To avoid these problems, venting is accomplished as the filter and support are fitted together, by the placement of three small protrusions (one of which is shown as 30ʹ) around the circumference of the interior of the outside wall 32ʹ of the filter holder 12ʹ. These protrusions raise the outer wall 32ʹ away from the exterior of the solid support (see arrow A) until contact is made by the respective annular groove and ridge. Air is thus allowed to exit the space between the filter holder and solid support until contact is made.

The salt (not shown) is provided in any suitable container, e.g. a cylindrical PVC container, preferably as a buffer solution. The buffer is hypertonic, preferably with an ionic strength greater than 0.5 M. In order to reduce the background level of reactivity of the assay membrane with blood solutions, the buffer also contains proteins unreactive with the assay membrane, for example, bovine serum albumin and non-fat dry milk. Preferably, the buffer also contains nonionic detergents, e.g., Tween® 80 or Triton® X-100 which increase the sensitivity of the assay.

### Assay

In the first step of the assay, filter 14 is wetted with buffer. Whole blood from the animal or human patient to be tested (approximately 3 drops (0.1 ml)) is gently mixed with 0.5 ml of buffer and the resulting treated whole blood solution is poured into the assembled membrane filter assembly having filter 14 in contact with assay membrane 18. The red blood cells in the blood are retained by the filter, and the red cell-free liquid containing the component to be detected passes through the filter to the assay membrane. After the filtration process, which takes approximately 1-5 minutes, is complete the filter assembly 10 is removed from the assay membrane and the assay membrane is processed using standard technique to detect the blood component being assayed.

The following example is illustrative.

### Example: Detection of Feline Leukemia Antigen

Antibodies to feline leukemia antigen (commercially available) are bound to latex microspheres by standard techniques and then spotted onto an assay membrane (Gelman AE filter) fixed to a membrane holder as in the Figure. A Gelman AE filter is mounted above and in contact with membrane 18, as shown in the Figure.

Blood is drawn from a cat by a standard procedure and 3 drops (approximately 0.1 ml) mixed with 0.5 ml of a buffer containing 0.25 molar sodium citrate, one molar KCl, 5% bovine serum albumin, 2.5% non-fat dry milk, and 0.1% Tween® 80. The resulting dilute whole blood solution is then poured into the assembly and given 3 minutes to pass through the filter onto the assay membrane. The filter assembly is then removed and the assay membrane treated to determine the presence or absence of feline leukemia antigen using conventional techniques. Generally, after the leukem ia antigen has bound to anti-leukemia antibody on the membrane, enzyme-labeled anti-leukemia antibody is added to form a "sandwich", and color is developed using an enzyme substrate and colormetric indicator.

Other embodiments are within the following claims. For example, although it is preferred that the red cell-free liquid be collected on a solid support as described above, the liquid can also be collected in a container and assayed using, e.g., a homogeneous assay method. Any hypertonic solution can be used which causes the required change in shape and deformability of the red cells in the blood, e.g., other salts, and sugar solutions. Any member of a specific binding pair can be measured, for example the assay can be used to screen whole blood samples for HTLV-3 virus or antibodies to this virus to test for Acquired Immune Deficiency Syndrome (AIDS).

## Claims

1. **Method of separating**
**red blood cells from a whole blood sample**
- to yield a sample fraction comprising plasma
- in preparation for
**assaying** the resulting sample fraction for
**one member of a specific binding pair**,
*characterized by*
- **treating** said sample **with** a **salt**
- so as to render said sample
- **hypertonic** and thereby to alter
- the **red blood cells** in said sample
- to **decrease** their ability to **pass** through **filter** media,
- **exposing** the resultant sample to
- a **filter** (14; 14')
- which **retains** said **red blood cells**
- but **allows** the passage **therethrough** of **filtrate** of said sample
- containing said **member** of said specific binding pair
- for **measuring** said **member** in the **filtrate**
*(Figs. 1;2).*

2. Method of claim 1 ,
*characterized in that*
- said **treating** said sample **with** said **salt**
- comprises
- **mixing** said sample with
- a **hypertonic solution**
- having
- an **ionic strength** of at least **0.5 M**.

3. Method of claim 1 ,
*characterized in that*
- a **non-ionic detergent**
- is **added** to said sample
- **prior** to **filtering**.

4. **Method of assaying**
a **filtered blood sample fraction** comprising plasma,
yielded by ay preceding claim, for
**one member of a specific binding pair**,
*characterized by*
- following
- exposing of said sample to **filter** (14; 14'),
- **depositing** said **filtrate** on
- a **solid support** (24, 18, 19, 21)
- on which
- **measuring** of said member of said specific binding pair
- is carried out
*(Figs. 1; 2).*

5. Method of claim 4 ,
*characterized in that*
- said **solid support** (24, 18, 19, 21) and
- said **filter** (14; 14')
- are in **contact** with **one another**
- when said sample is exposed to said **filte**r (14; 14').

6. Method of any preceding claim ,
*characterized in that*
- **one member** of said **specific binding pair** is
- a **virus** or
- an **antibody against** a **virus**.

7. Method of claim 6,
*characterized in that*
- said **virus** is
- **HTLV-3**.

8. **Method of assaying**
a **sample** of **whole blood** for
**feline leukemia antigen**,
*characterized by*
- **treating** said sample **with** a **salt**
- so as to render said sample
- **hypertonic** and thereby to alter
- the **red blood cells** in said sample
- to **decrease** their ability to **pass** through **filter** media,
- **exposing** the resultant sample to
- a **filter** (14; 14')
- which **retains** said **red blood cells**
- but **allows** the passage **therethrough** of **filtrate** of said sample
- containing said **antigen**, and
- **measuring** said **antigen** in the **filtrate**
*(Figs. 1; 2)*.

9. Method of claim 8 ,
*characterized in that*
- said **treating** said sample **with** said **salt**
- comprises
- **mixing** said sample with
- a **hypertonic solution**
- having
- an **ionic strength** of at least **0.5 M**, so as to render said sample hypertonic.

10. Method of claim 9 ,
*characterized in that*
- said **hypertonic solution**
- comprises
- **KCl** and
- **sodium citrate**.

11. Method of claim 10 ,
*characterized in that*
- said **hypertonic solution**
- comprises
- **nonionic detergent**.

12. Method of claim 11 ,
*characterized in that*
- said **hypertonic solution**
- comprises
- **bovine serum albumin** and
- **non-fat dry milk**.

13. **Method of assaying**
a **filtered blood sample fraction** comprising plasma ,
yielded by any of claims 1 - 3 , for
**one member of a specific binding pair**,
*characterized in that*
- **filtration** is accomplished in
- a **filtration device**
- comprising
- a **filter** (14; 14'), and
- said **filtrate**
- is **removed** from said **filtration device** and
- placed in **contact** with
- a **measuring device**
- comprising
- a **solid support** (24, 18, 19, 21)
- on which said measuring of said member of said specific binding pair
- is carried out,
- said **filter** (14; 14') and
- said **support**
- being **separated** from **each other**
*(Figs. 1; 2).*

14. **Kit for assaying**
a **sample** of **whole blood** for
**one member of a specific binding pair**,
*characterized by*
- a **first container**
- **holding** a **hypertonic solution**,
- a **second container** for
- **holding** said **hypertonic solution** and
- **mixing** said **hypertonic solution** with said **sample** ,
- a **filter** (14; 14')
- positioned with respect to said second container
- so as to permit said mixture of said sample and said hypertonic solution
- to pass from said second container through said filter (14; 14'),
- said filter (14; 14')
- being capable of retaining the treated red blood cells in said sample, and
- **means** for **collecting** said **sample**
- after it has passed through said filter (14; 14')
*(Figs. 1; 2).*

15. Kit of claim 14 ,
*characterized in that*
- said **collecting means**
- comprises
- a **solid support** (24, 18, 19, 21).

16. Kit of claim 15,
*characterized in that*
- said **filter** (14; 14') is
- **flexible** , and
- said **kit**
- comprises
- **means** to **hold** said filter (14; 14')
- in intimate contact with said **solid support** (24, 18, 19, 21).

17. Kit of claim 15 ,
*characterized in that*
- said **solid support** (24, 18, 19, 21)
- comprises
- a **surface**
- **bound** to **one** of the **components** of said **specific binding pair**.

18. Kit of claim 17 ,
*characterized in that*
- said **one member** of said **specific binding pair** is
- an **antigen** ,
- a **virus** , or
- an **antibody** .

19. Kit of claim 14 ,
*characterized in that*
- said **filter** (14; 14')
- is positioned within
- a **holder** (12; 12').

20. Kit of claim 19 ,
*characterized in that*
- said **holder** (12; 12')
- comprises
- a **fluid chamber** (11)
- having
- **sloping sides**
- to improve flow consistency.

21. Kit of claim 19 ,
*characterized in that*
- said **filter** (14; 14')
- is **sealed** to said **holder** (12; 12').

22. Kit of claim 19 ,
*characterized by*
- a **measuring device** ,
- said **filter holder** (12; 12')
- comprising
- an **annular member**
- configured
- to seat over said measuring device.

23. Kit of claim 22 ,
*characterized in that*
- said **annular member**
- comprises
- an **annular exterior wall** (32') and
- an **annular interior wall** (38') ,
- attached by
- a **connecting member** ,
- said **exterior wall** (32') and said **interior wall** (38')
- being **spaced** apart a distance
- selected to form a **cavity**
- for receiving
- a co-operatively shaped
- **seating member**
- connected to
- said **measuring device** ,
- to insure intimate **contact** between
- said filter (14; 14') and said solid support (24, 18, 19, 21)
*(Fig. 2)*.

24. Kit of claim 23 ,
*characterized in that*
- at least **one** of
- said **interior wall** (38') and
- said **exterior wall** (32')
- comprises
- a **surface irregularity** (30')
- along said cavity
- to seat said **measuring device**
*(Fig. 2)*.

25. Kit of claim 24 ,
*characterized in that*
- said **surface irregularity**
- comprises
- **means** (30') for **venting air**
- as said filter device (10; 10')
- is seated on said **measuring device**
*(Fig. 2)*.

26. Kit of claim 14 ,
*characterized by*
- a **means** for **reducing non-specific binding**.

27. **Filter device** (10; 10')
*characterized by*
- a **filter** (14; 14') and
- a **filter holder** (12; 12'),
- said **filter holder** (12; 12')
- comprising
- an **annular member**
- configured
- to seat over
- a **solid-phase assay member** (24, 18, 19, 21), and
- **annular means**
- comprising
- an **annular exterior wall** (32') and
- an **annular interior wall** (38'),
- attached by
- a **connecting member** ,
- said **exterior wall** (32') and
- said **interior wall** (38')
- being **spaced** apart a distance selected to form
- a **cavity**
- sized and shaped for receiving
- a co-operatively shaped
- **measuring device**
*(Figs. 1; 2)*.

28. Filter device of claim 27 ,
*characterized in that*
- at least **one** of
- said **interior wall** (38') and
- said **exterior wall** (32')
- comprises
- a **surface irregularity** (30')
- along said cavity
- to seat said **measuring device** (24, 18, 19, 21)
*(Fig. 2)*.

29. Filter device of claim 28 ,
*characterized in that*
- said **surface irregularity**
- comprises
- **means** (30') for **venting air**
- as said filter device (10; 10')
- is seated on said **measuring device** (24, 18, 19, 21)
*(Fig. 2)*.

30. Filter device of claim 27 ,
*characterized in that*
- said **filter holder** (12; 12')
- comprises
- **means** (39') to impart **tension to** said **filter** (14; 14')
- to insure intimate **contact** between
- said filter (14; 14') and a measuring device (24, 18, 19, 21)
*(Fig. 2)*.

31. Filter device of claim 30 ,
*characterized in that*
- said **means** to impart **tension**
- comprises
- a **screen** (39')
- positioned to contact the filter (14; 14')
*(Fig. 2)*.

32. Filter device of claim 31 ,
*characterized in that*
- said **screen** (39') is
- **annular**
*(Fig. 2)*.

## Patentansprüche

1. Verfahren zum Separieren roter Blutzellen aus einer Vollblutprobe, um einen Probenanteil, der Plasma aufweist, zur Vorbereitung der Untersuchung des resultierenden Probenanteils auf eines der Glieder eines spezifischen Bindungspaares zu gewinnen, gekennzeichnet durch
- Behandeln der Probe mit Salz, so daß die Probe hypertonisch wird und dadurch die roten Blutzellen in der Probe verändert werden, um ihre Fähigkeit zu vermindern, Filtermedien zu passieren;
- Aussetzen der resultierenden Probe der Einwirkung eines Filters (14; 14'), das die roten Blutzellen zurückhält, aber den Durchtritt von Filtrat der Probe ermöglicht, das das genannte Glied des spezifischen Bindungspaares enthält, um das Glied im Filtrat zu messen (Fig. 1; 28)

2. Verfahren nach Anspruch 1,
dadurch gekennzeichnet,
daß die Behandlung der Probe mit Salz das Mischen der Probe mit einer hypertonischen Lösung umfaßt, die eine Ionenstärke von mindestens 0,5 M hat.

3. Verfahren nach Anspruch 1,
dadurch gekennzeichnet,
daß der Probe vor dem Filtern ein nichtionisches Detergens beigegeben wird.

4. Verfahren zum Untersuchen eines gefilterten Blutprobenanteils, der Plasma aufweist und durch einen beliebigen vorhergehenden Anspruch gewonnen wird, auf eines der Glieder eines spezifischen Bindungspaares,
gekennzeichnet durch
Auftragen des Filtrates, im Anschluß an das Untersuchen der Probe der Wirkung des Filters, auf einen festen Träger (24, 18, 19, 21), auf dem das Messen des genannten Gliedes des spezifischen Bindungspaares durchgeführt wird (Fig. 1; 2)

5. Verfahren nach Anspruch 4,
dadurch gekennzeichnet,
daß der feste Träger (24, 18, 19, 21) und das Filter (14; 14') dargeboten wird (14; 14').

6. Verfahren nach einem beliebigen vorhergehenden Anspruch,
dadurch gekennzeichnet,
daß eines der Glieder des spezifischen Bindungspaares ein Virus oder ein gegen ein Virus wirkender Antikörper ist.

7. Verfahren nach Anspruch 6, dadurch gekennzeichnet,
daß das Virus HTLV-3 ist.

8. Verfahren zum Untersuchen einer Probe Vollblut auf ein Katzenleukämieantigen,
gekennzeichnet durch
- Behandeln der Probe mit Salz, so daß die Probe hypertonisch wird und dadurch die roten Blutzellen in der Probe verändert werden, um ihre Fähigkeit zu vermindern, Filtermedien zu passieren;
- Aussetzen der resultierenden Probe der Einwirkung eines Filters (14; 14'), das die roten Blutzellen zurückhält, aber den Durchtritt von Filtrat der Probe ermöglicht, das das genannte Antigen enthält; und
- Messen des Antigens im Filtrat.

9. Verfahren nach Anspruch 8,
dadurch gekennzeichnet,
daß die Behandlung der Probe mit Salz das Mischen der Probe mit einer hypertonischen Lösung umfaßt, die eine Ionenstärke von mindestens 0,5 M hat, so daß die Probe hypertonisch wird.

10. Verfahren nach Anspruch 9,
dadurch gekennzeichnet,
daß die hypertonische Lösung KCl und Natriumnitrat aufweist.

11. Verfahren nach Anspruch 10,
dadurch gekennzeichnet,
daß die hypertonische Lösung ein nichtionisches Detergens aufweist.

12. Verfahren nach Anspruch 11,
dadurch gekennzeichnet,
daß die hypertonische Lösung das Rinderserum Albumin und magere Trockenmilch aufweist.

13. Verfahren zum Untersuchen eines gefilterten Blutprobenanteils, der Plasma aufweist und durch einen beliebigen Anspruch 1 - 3 gewonnen wird, auf eines der Glieder eines spezifischen Bindungspaares,
dadurch gekennezeichnet,
daß das Filtern in einer Filtereinrichtung mit einem Filter (14, 14') durchgeführt wird, und das Filtrat aus der Filtereinrichtung entfernt und in Berührung mit einer Meßeinrichtung gebracht wird, die einen festen Träger (24, 18, 19, 21) aufweist, auf dem die Messung des genannten Gliedes des spezifischen Bindungspaares durchgeführt wird, wobei der Filter (14, 14') und der Träger voneinander getrennt werden (Fig. 1; 2).

14. Ausrüstungssatz zum Untersuchen einer Vollblutprobe auf eines der Glieder eines spezifischen Bindungspaares, gekennzeichnet durch
- einen ersten Behälter, der eine hypertonische Lösung enthält,
- einen zweiten Behälter zur Aufnahme der hypertonischen Lösung und zum Mischen der hypertonischen Lösung mit der Probe,
- ein Filter (14; 14')
das in Bezug auf den zweiten Behälter so positioniert ist, daß die Mischung bestehend aus der genannten Probe und der hypertonischen Lösung vom zweiten Behälter her durch das Filter (14; 14') hindurchtreten kann, wobei das Filter in der Lage ist, die behandeltern roten Blutzellen in der Probe zurückzuhalten, und
- Einrichtungen zum Sammeln der Probe, nachdem sie das Filter (14; 14') passiert hat (Fig. 1; 2).

15. Ausrüstungssatz nach Anspruch 14,
dadurch gekennzeichnet,
daß die Sammeleinrichtungen einen festen Träger (24, 18, 19, 21) umfassen.

16. Ausrüstungssatz nach Anspruch 15,
dadurch gekennzeichnet,
daß das Filter (14; 14') flexibel ist, und der Ansrüstungssatz Einrichtungen zum Halten des Filters (14; 14') in engstem Kontakt dem festen Träger (24, 18, 19, 21) aufweist.

17. Ansrüstungssatz nach Anspruch 15,
dadurch gekennzeichnet,
daß der feste Träger (24, 18, 19, 21) eine Oberfläche aufweist, die an eine der Komponenten des spezifischen Bindungspaares gebunden ist.

18. Ausrüstungssatz nach Anspruch 17,
dadurch gekennzeichnet,
daß das eine Glied des spezifischen Bindungspaares ein Antigen, ein Virus oder ein Antikörper ist.

19. Ausrüstungssatz nach Anspruch 14,
dadurch gekennzeichnet,
daß das Filter (14; 14') in einem Halter (12; 12') positioniert ist.

20. Ausrüstungssatz nach Anspruch 19,
dadurch gekennzeichnet,
daß der Halter (12; 12') eine Fluidkammer (11) mit schrägen Seiten aufweist, um die Fließkonsistenz zu verbessern.

21. Ausrüstungssatz nach Anspruch 19,
dadurch gekennzeichnet,
daß das Filter (14; 14') dicht mit dem Halter (12; 12') verbunden ist.

22. Ausrüstungssatz nach Anspruch 19,
gekennzeichnet durch eine Meßeinrichtung, wobei der Filterhalter (12; 12') ein ringförmiges Element aufweist, daß zum Sitzen über der Meßeinrichtung ausgebildet ist.

23. Ausrüstungssatz nach Anspruch 22,
dadurch gekennzeichnet,
daß das ringförmige Element eine ringförmige Außenwand (32') und eine ringförmige Innenwand (38') aufweist, die durch ein Verbindungsglied befestigt sind, wobei die Außenwand (32') und die Innenwand (38') einen gegenseitigen Abstand einnehmen, der so gewählt ist, daß er einen Hohlraum zur Aufnahme eines kooperativ geformten Sitzelelmentes bildet, das mit der Meßeinrichtung verbunden ist, um engsten Kontakt zwischen dem Filter (14; 14') und dem festen Träger (24, 18, 19, 21) zu gewährleisten.

24. Ausrüstungssatz nach Anspruch 23,
dadurch gekennzeichnet,
daß von der Innenwand (38') und der Außenwand (32) mindestens eine Wand eine Oberflächenunregelmäßigkeit (30') entlang des Hohlraumes aufweist, um die Meßeinrichtung aufzusetzen.

25. Ausrüstungssatz nach Anspruch 24,
dadurch gekennzeichnet,
daß die Oberflächenunregelmäßigkeit eine Einrichtung (30') zur Belüftung aufweist, wenn die Filtereinrichtung auf der Meßeinrichtung aufsitzt (Fig. 2).

26. Ausrüstungssatz nach Anspruch 14,
gekennzeichnet durch
ein Mittel zur Verringerung der nichtspezifischen Bindung.

27. Filtereinrichtung (10; 10')
gekennzeichnet durch
ein Filter (14; 14') und einen Filterhalter (12; 12'), wobei der Filterhalter (12; 12') aufweist:
- ein ringförmiges Element, daß zum Sitzen über einer Festphasenuntersuchungselement (24, 18, 19, 21) ausgebildet ist, und
- ringförmige Einrichtungen, die eine ringförmige Außenwand (32') und eine ringförmige Innenwand (38') aufweisen, die durch ein Verbindungsglied befestigt sind; wobei die Außenwand (32') und die Innenwand (38') einen gegenseitigen Abstand einnehmen, der so gewählt ist, daß ein Hohlraum zum Aufnehmen einer kooperativ gestalteten Meßeinrichtung gebildet wird (Fig. 1; 2).

28. Filtereinrichtung nach Anspruch 27,
dadurch gekennzeichnet,
daß von der Innenwand (38') und der Außenwand (32') mindestens eine Oberflächenunregelmäßigkeit (30') entlang des Hohlraumes aufweist, um die Meßeinrichtung aufzusetzen (24, 18, 19, 21)

29. Filtereinrichtung nach Anspruch 28,
dadurch gekennzeichnet,
daß die Oberflächenunregelmäßigkeit eine Einrichtung (30') zur Belüftung aufweist, wenn die Filtereinrichtung auf der Meßeinrichtung aufgesetzt ist (Fig. 2) (24, 18, 19, 21).

30. Filtereinrichtung nach Anspruch 27,
dadurch gekennzeichnet,
daß der Filterhalter (12, 12') eine Eirichtung (39') zum Aufbringen von Spannung auf das Filter (14; 14') aufweist, um engsten Kontakt zwischen dem Filter (14, 14') und einer Meßeinrichtung (24, 18, 19, 21) zu gewährleisten (Fig. 2).

31. Filtereinrichtung nach Anspruch 30,
dadurch gekennzeichnet,
daß die Einrichtung zuum Aufbringen von Spannung ein Sieb (39') umfaßt, das für die Kontaktaufnahme mit dem Filter positioniert ist. (Fig. 2).

32. Filtereinrichtung nach Anspruch 31,
dadurch gekennzeichnet,
daß das Sieb (39') ringförmig ist (Fig. 2).

## Revendications

1. **Procédé pour séparer**
**les globules rouges d'un échantillon de sang entier**
- pour donner une fraction échantillon comprenant du plasma
- en préparation pour
**doser** dans la fraction échantillon résultante
**l'un des éléments d'une paire liante spécifique**,
***caractérisé** par les étapes consistant à :*
- **traiter** cet échantillon **avec** un **sel**
- afin de rendre cet échantillon
- **hypertonique** et ainsi altérer
- les **globules rouges du sang** de l'échantillon
- pour **réduire** leur aptitude à **traverser** le milieu **filtrant**,
- **exposer** l'échantillon résultant à
- un **filtre** (14; 14')
- qui **retient** lesdits **globules rouges du sang**
- mais **autorise** le passage **au travers** d'un **filtrat** dudit échantillon
- contenant ledit **élément** de ladite paire liante spécifique
- pour la **mesure** de cet **élément** dans le **filtrat**
(*figures 1 ; 2)*

2. Procédé de la revendication 1,
***caractérisé** en ce que*
- le **traitement** de l'échantillon **par** le **sel**
- comprend
- le **mélange** de l'échantillon avec
- une solution **hypertonique**
- présentant
- une **force ionique** d'au moins **0,5 M**.

3. Procédé de la revendication 1,
***caractérisé** en ce que*
- l'on **ajoute** à l'échantillon,
- **avant** le **filtrage**
- un **détergent non ionique**.

4. **Procédé pour doser**
dans une **fraction d'échantillon de sang filtré** comprenant du plasma,
obtenue par l'une quelconque des revendications précédentes,
**l'un des éléments d'une paire liante spécifique**,
***caractérisé** en ce que*
- après
- avoir exposé l'échantillon au **filtre** (14 ; 14'),
- on **dépose** ledit **filtrat** sur
- un **support solide** (24, 18, 19, 21) sur lequel
- on exécute
- une mesure dudit élément de ladite paire liante spécifique.
*(figures 1 ; 2)*

5. Procédé de la revendication 4,
***caractérisé** en ce que*
- le **support solide** (24, 18, 19, 21) et
- le **filtre** (14 ; 14')
- sont en **contact** l'**un avec l'autre**
- lorsque l'échantillon est exposé au **filtre** (14 ; 14').

6. Procédé selon l'une des revendications précédentes,
***caractérisé** en ce que*
- l'un des **éléments** de ladite **paire liante spécifique** est
- un **virus** ou
- un **anticorps contre** un **virus**.

7. Procédé de la revendication 6,
***caractérise** en ce que*
- ledit **virus** est
- **HTLV-3**.

8. **Procédé pour doser**
dans un **échantillon** de **sang entier**
**l'antigène de la leucémie féline**,
***caractérisé** par les étapes consistant à :*
- **traiter** cet échantillon **avec** un **sel**
- afin de rendre cet échantillon
- **hypertonique** et ainsi altérer
- les **globules rouges du sang** de l'échantillon
- pour **réduire** leur aptitude à **traverser** le milieu **filtrant**,
- **exposer** l'échantillon résultant à
- un **filtre** (14 ; 14')
- qui **retient** lesdits **globules rouges du sang**
- mais **autorise** le passage **au travers** d'un **filtrat** dudit échantillon
- contenant ledit **élément** de ladite paire liante spécifique
- pour la **mesure** de cet **élément** dans le **filtrat**
*(figures 1 ; 2)*

9. Procédé selon la revendication 8,
***caractérisé** en ce que*
- le **traitement** de l'échantillon **par** le **sel**
- comprend
- le **mélange** de l'échantillon avec
- une solution **hypertonique**
- présentant
- une **force ionique** d'au moins **0,5 M**. de manière à rendre hypertonique cet échantillon.

10. Procédé selon la revendication 9,
***caractérisé** en ce que*
- la **solution hypertonique**
- comprend
- **KCl** et
- du **citrate de sodium**.

11. Procédé selon la revendication 10,
***caractérisé** en ce que*
- la **solution hypertonique**
- comprend
- un **détergent non ionique**.

12. Procédé selon la revendication 11,
***caractérisé** en ce que*
- la **solution hypertonique**
- comprend
- de la **sérum albumine bovine** et
- du **lait desséché écrémé**.

13. **Procédé de dosage**
dans une **fraction d'échantillon de sang filtré** comprenant du plasma,
obtenue par l'une quelconque des revendications 1 à 3,
d**'un élément d'une paire liante particulière**,
***caractérisé** en ce que*
- la **filtration** est réalisée par
- un **dispositif de filtration**
- comprenant
- un **filtre** (14 ; 14') et
- le **filtrat**
- est **retiré** de l'**appareil de filtration** et
- placé en **contact** avec
- un **dispositif de mesure**
- comprenant
- un **support solide** (24, 18, 19, 21)
- sur lequel la mesure de l'élément de la paire liante spécifique
- est effectuée,
- le **filtre** (14 ; 14') et
- le **support**
- étant **distincts** l'**un de l'autre**.
*(figures 1 ; 2)*

14. **Nécessaire de dosage**
dans un **échantillon** de **sang entier**
d'**un élément d'une paire liante spécifique**,
***caractérisé** par*
- un **premier récipient**
- **enfermant** une **solution hypertonique**,
- un **second récipient**, pour
- **enfermer** la **solution hypertonique** et
- **mélanger** cette **solution hypertonique** avec l'**échantillon**,
- un **filtre** (14 ; 14')
- positionné par rapport au second récipient
- de manière à permettre au mélange de l'échantillon et de la solution hypertonique
- de passer du second récipient jusqu'au travers du filtre (14 ; 14'),
- le filtre (14 ; 14')
- étant capable de retenir les globules rouges du sang traités de l'échantillon, et
- des **moyens** pour **recueillir** l'**échantillon**
- après qu'il ait traversé le filtre (14 ; 14')
*(figures 1 ; 2)*

15. Nécessaire selon la revendication 14,
***caractérisé** en ce que*
- les **moyens de recueil**
- comprennent
- un **support solide** (24, 18, 19, 21)

16. Nécessaire selon la revendication 15
***caractérisé** en ce que*
- le **filtre** (14 ; 14') est
- **flexible** et
- le **nécessaire**
- comprend
- des **moyens** pour **maintenir** ce filtre (14 ; 14')
- en contact intime avec le **support solide** (24, 18, 19, 21).

17. Nécessaire selon la revendication 15,
***caractérisé** en ce que*
- le **support solide** (24, 18, 19, 21)
- comprend
- une **surface**
- **liée** à l'**une** des **composantes** de la **paire liante spécifique.**

18. Nécessaire selon la revendication 17,
***caractérisé** en ce que*
- le **premier élément** de la **paire liante spécifique** est
- un **antigène**,
- un **virus** ou
- un **anticorps**.

19. Nécessaire selon la revendication 14,
***caractérisé** en ce que*
- le **filtre** (14 ; 14')
- est disposé à l'intérieur
- d'un **porte-filtre** (12 ; 12').

20. Nécessaire selon la revendication 19,
***caractérisé** en ce que*
- le **porte-filtre** (12 ; 12')
- comprend
- une **chambre à fluide** (11)
- possédant
- des **côtés en pente**
- afin d'améliorer l'homogénéité d'écoulement.

21. Nécessaire selon la revendication 19,
***caractérisé** en ce que*
- le **filtre** (14 ; 14')
- est **scellé** au **porte-filtre** (12 ; 12').

22. Nécessaire selon la revendication 19,
***caractérisé** par*
- un **dispositif de mesure**,
- le **porte-filtre** (12 ; 12')
- comprenant
- une **partie annulaire**
- configurée
- de manière à venir se poser sur l'appareil de mesure.

23. Nécessaire selon la revendication 22,
***caractérisé** en ce que*
- la **partie annulaire**
- comprend
- une **paroi annulaire extérieure** (32) et
- une **paroi annulaire intérieure** (38')
- réunies par
- une **partie de liaison**,
- la **paroi extérieure** (32') et la **paroi intérieure** (38')
- étant **éloignées** l'une de l'autre d'une distance
- choisie de manière à former une **cavité**
- permettant de recevoir
- une **partie de dépose**
- de forme coopérante,
- reliée à
- l'**appareil de mesure**
- afin d'assurer un **contact** intime entre
- le filtre (14 ; 14') et le support solide (24, 18, 19, 21)
*(figure 2)*

24. Nécessaire selon la revendication 23,
***caractérisé** en ce que*
- l'**une** au moins de
- la **paroi intérieure** (38') et
- la **paroi extérieure** (32')
- comprend
- une **irrégularité de surface** (30')
- le long de ladite cavité
- venant se poser sur le **dispositif de mesure**.
*(figure 2)*

25. Nécessaire selon la revendication 24,
***caractérise** en ce que*
- l'**irrégularité de surface**
- comprend
- des **moyens** (30') pour **éventer l'air**
- lorsque le dispositif filtrant (10 ; 10')
- est déposé sur le **dispositif de mesure**.
*(figure 2)*

26. Nécessaire selon la revendication 14,
***caractérisé** par*
- un **moyen** pour **réduire la liaison non spécifique**.

27. **Dispositif filtrant** (10 ; 10')
***caractérisé** par*
- un **filtre** (14 ; 14') et
- un **porte-filtre** (12 ; 12'),
- ce **porte-filtre** (12 ; 12')
- comprenant
- une **partie annulaire**
- configurée
- de manière à reposer sur
- une **partie de dosage en phase solide** (24, 18, 19, 21) et
- des **moyens annulaires**
- comprenant
- une **paroi annulaire extérieure** (32') et
- une **paroi annulaire intérieure** (38')
- réunies par
- une **partie de liaison**
- la **paroi extérieure** (32') et
- la **paroi intérieure** (38')
- étant **éloignées** l'une de l'autre d'une distance choisie de manière à former
- une **cavité**
- dimensionnée et conformée de manière à recevoir
- un **dispositif de mesure**
- de forme coopérante.
*(figures 1 ; 2)*

28. Dispositif filtrant selon la revendication 27,
***caractérisé** en ce que*
- l'**une** au moins de
- la **paroi intérieure** (38') et
- la **paroi extérieure** (32')
- comprend
- une **irrégularité de surface** (30')
- le long de ladite cavité
- venant se poser sur le **dispositif de mesure**.
*(figure 2)*

29. Dispositif filtrant selon la revendication 28,
***caractérisé** en ce que*
- l'**irrégularité de surface**
- comprend
- des **moyens** (30') pour **éventer l'air**
- lorsque le dispositif filtrant (10 ; 10')
- est déposé sur le **dispositif de mesure**.
*(figure 2)*

30. Dispositif filtrant selon la revendication 27,
***caractérisé** en ce que*
- le **porte-filtre** (12 ; 12')
- comprend
- des **moyens** (39') pour appliquer une **tension au filtre** (14 ; 14')
- afin d'assurer un **contact** intime entre
- ce filtre (14 ; 14') et un dispositif de mesure (24, 18, 19, 21).
*(figure 2)*

31. Dispositif filtrant selon la revendication 30,
***caractérisé** en ce que*
- les **moyens** pour appliquer une **tension**
- comprennent
- un **écran** (39')
- positionné de manière à venir en contact avec le filtre (14 ; 14').
*(figure 2)*

32. Dispositif filtrant selon la revendication 31,
***caractérisé** en ce que*
- l'**écran** (39') est
- **annulaire**.
*(figure 2)*
